# EUROPÄISCHE PATENTSCHRIFT

(11) **EP 2 440 571 B1**
(45) Veröffentlichungstag und Bekanntmachung des Hinweises auf die Patenterteilung: **26.02.2014**
(21) Anmeldenummer: 10724252.1
(22) Anmeldetag: 30.04.2010
(51) Int. Cl.: C07K 7/54, A01N 63/02, A61P 3/10, A61P 25/28, A61P 29/00, A61P 35/00

(54) **HERSTELLUNG UND VERWENDUNG ANTITUMORALER, ANTIBIOTISCHER UND INSEKTIZIDER CYCLODEPSIPEPTIDE**
PRODUCTION AND USE OF ANTITUMORAL, ANTIBIOTIC AND INSECTICIDAL CYCLODEPSIPEPTIDES
PRODUCTION ET UTILISATION DE DEPSIPEPTIDES CYCLIQUES ANTITUMORAUX, ANTIBIOTIQUES ET INSECTICIDES

(30) Priorität: 11.06.2009 DE 102009025119
(43) Veröffentlichungstag der Anmeldung: 18.04.2012
(73) Patentinhaber: Helmholtz-Zentrum für Ozeanforschung Kiel (GEOMAR), 24148 Kiel (DE)
(72) Erfinder: IMHOFF, Johannes, 24211 Preetz (DE); KNOPF-KAJAHN, Inga, 24214 Gettorf (DE); LANG, Gerhard, CH-1796 Courgevaux / Gurwolf (CH); WIESE, Jutta, 23701 Eutin (DE); PETERS, Arne, 24217 Höndorf (DE)
(74) Vertreter: UEXKÜLL & STOLBERG
(86) Internationale Anmeldenummer: PCT/DE2010/000498
(87) Internationale Veröffentlichungsnummer: WO 2010/142258

(56) Entgegenhaltungen:
- WO-A1-2005/034982
- DE-A1- 10 252 284

## Beschreibung

Die vorliegende Erfindung betrifft zyklische Peptide, die Xenobovide, die zur Herstellung von Medikamenten zur Behandlung von Infektions-, Tumor-, neurologischen und entzündungsbasierten Erkrankungen sowie von Diabetes geeignet sind. Weiteres Anwendungsgebiet der Erfindung ist die Herstellung von Insektiziden und Pflanzenschutzmitteln. Die Erfindung betrifft außerdem ein Verfahren zur Produktion dieser Verbindungen, durch Fermentation eines Bakteriums und chromatographische Aufreinigung des Bakterienextraktes, sowie deren Verwendung als Arzneimittel, Insektizid und Pflanzenschutzmittel.

Bestimmte Genera von Nematoden enthalten insektentötende bakterielle Symbionten. Die im Boden lebenden Nematoden spüren in der Natur Insekten auf und stechen die Oberfläche des Insekts durch, so dass pathogene Bakterien in das Hämozöl des Insekts injiziert werden. Diese Bakterien produzieren Antibiotika, Enzyme und Toxine, die das Insekt töten. Die im Insekt vorhandenen Nematoden vermehren sich, erwerben weitere Bakterien und werden aus dem sich zersetzenden Kadaver freigesetzt, um neue Insekte aufzusuchen.

Die Bakterien der Gattung *Xenorhabdus,* mit *Steinernema*-Nematoden symbiotisch assoziiert, sind bekannt als Quelle einer großen Anzahl biologisch aktiver Substanzen. Aus diesen entomopathogenen Bakterien wurden verschiedene Proteine isoliert, die für Insekten toxisch sind. Dies ist z.B. der Fall für TC-Proteine ("toxin complex proteins") wie in der US 7285632 B2 oder in der US 2008/0104731 A1 beschrieben, für "TC-like"-Proteine (EP 1130970 B1, EP 1143800 B1), oder für eine Reihe anderer Toxinproteine (US 6048838, US 6174860 B1, US 6277823 B1, US 7214525 B1, US 6841165 B1). Mehrere aus diesen Bakterien isolierten antibakteriell wirkende Substanzen sind ebenfalls bekannt: Pseudopeptide (EP 1351977 B1), das Peptid Xenorhabdicin (Thaler et al. 1995, Purification and characterization of xenorhabdicin, a phage tail-like bacteriocin, from the lysogenic strain F1 of Xenorhabdus nematophilus. Appl. Environ. Microbiol. 61:2049-2052), die Xenorhabdine (EP 0192713 B1), die Xenocoumacine (EP 0192713 B1), die Nematophine (US 5569668), die Xenomine (US 5827872), oder die Xenorxide (US 6316476 B1). Xenomine und Xenorxide weisen zudem antineoplasische Aktivität auf (US 6583171 B1).

DE 102 52 284 A1 und WO 2005/034982 A1 offenbaren Cyclopeptide mit pharmakologischen, herbiziden und/oder insektiziden Eigenschaften.

Weltweit besteht ein zunehmender Bedarf an neuen Wirkstoffen für die Behandlung von Infektions-, Krebs- und Entzündungserkrankungen sowie von Diabetes und neurologische Krankheiten. Des Weiteren besteht ein Bedarf an neuen Substanzen, die als Insektizide bzw. Pflanzenschutzmittel eingesetzt werden können.

Damit liegt der vorliegenden Erfindung die Aufgabe zugrunde, weitere Peptide bereitzustellen, die antibiotisch, antitumoral, entzündungshemmend, Blutzuckerspiegel senkend, Acetylcholinspiegel steigernd oder insektizid wirksam sind oder die Aktivität der Acetylcholinesterase, Phosphodiesterase, Protein Tyrosin Phosphatase oder der Glycogen-Synthase-Kinase hemmen, oder gegen Erreger von Pflanzenkrankheiten wirksam sind, sowie einen Weg zu ihrer Produktion aufzuzeigen.

Erfindungsgemäß wird diese Aufgabe durch die Verbindung mit den Merkmalen des Anspruch 1 gelöst. Die Unteransprüche 2-15 geben vorteilhafte Ausführungen der Erfindung wieder.

Innerhalb eines wissenschaftlichen Programms zur Isolierung von biologisch aktiven natürlichen Produkten aus Mikroorganismen haben die Erfinder das Bakterium *Xenorhabdus bovienii* aus dem Nematoden *Steinernema bibionis* isoliert und untersucht.

Durch Fast Centrifugal Partitioning Chromatography (FCPC) und präparative HPLC wurden aus Kulturen des Bakteriums *Xenorhabdus bovienii* die erfindungsgemäß bevorzugten Peptide Xenobovid A, B und C isoliert.

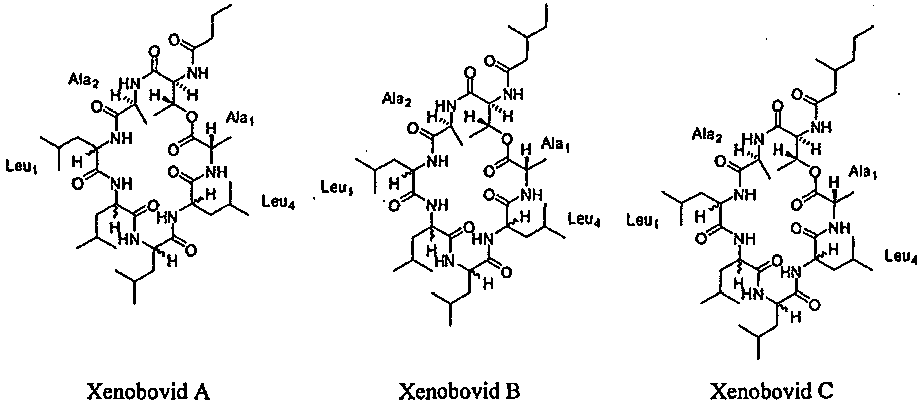

Die physikalischen Daten von Xenobovid A, B und C sind in den beigefügten Tabellen 1, 2 und 3 dargestellt, wobei Tabelle 1 die NMR-Daten (600 MHz, DMSO) von Xenobovid A, Tabelle 2 die NMR-Daten (600 MHz, DMSO) von Xenobovid B und Tabelle 3 die NMR-Daten (600 MHz, DMSO) von Xenobovid C zeigen.

Die erfindungsgemäßen Substanzen haben in den spezifischen Ausführungsformen Xenobovid A, B und C die Fähigkeit, das Wachstum des Gram-positiven Bakteriums *Staphylococcus xylosus* zu hemmen. Xenobovid B und C weisen zudem eine signifikante Hemmwirkung gegen das Gram-positive Bakterium *Bacillus subtilis* (Beispiel 3).

Das Wachstum des phytopathogenen Bakteriums *Xanthomonas campestris,* u.a. Erreger der Adernschwärze bei Kohl, wird von Xenobovid A gehemmt (Beispiel 4).

Die erfindungsgemäßen Substanzen Xenobovid A, B und C zeigen außerdem unterschiedliche, signifikante Hemmwirkungen gegen Tumorzelllinien, insbesondere gegen humanen Magentumor-, Lungentumor-, Brustkrebs-, Melanom-, Pankreastumor-, Nierentumor- und Darmtumor-Zelllinien (s. Beispiel 5).

Xenobovid A hemmt die Aktivität folgender Enzyme:
- Acetylcholinesterase (Beispiel 6): Inhibitoren des Abbaus von Acetylcholin können in der Behandlung bzw. Prävention neurologischer Erkrankungen wie Myasthenia Gravis oder Alzheimer eingesetzt werden.
- Phosphodiesterase PDE-4B2 (Beispiel 7): PDE4-Ininhibitoren sind erprobte antiinflammatorische Mittel, insbesondere bei entzündlichen Lungenerkrankungen wie Asthma, chronisch obstruktive Bronchitis und Lungenemphysem, sowie bei Rhinitis. Diese Inhibitoren können zudem eine antidepressive Wirkung aufweisen und wurden auch zur Verwendung als Psychopharmaka vorgeschlagen. Darüber hinaus sind PDE4-Ininhibitoren in der Lage, die Virulenz von HIV abzuschwächen und könnten somit in der AIDS-Therapie eingesetzt werden.
- Protein Tyrosin Phosphatase 1B (Beispiel 8): Protein Tyrosin Phosphatase 1B ist ein effektives Ziel für die Behandlung von Diabetes und Fettleibigkeit.

Sowohl Xenobovid A als auch Xenobovid B hemmen zudem die Aktivität der Glycogen Synthase Kinase 3beta (Beispiel 9). Die Inhibition dieses Enzyms kann in der Behandlung von Alzheimer und anderen akuten oder chronischen neurodegenerative Erkrankungen, sowie von Diabetes und Fettleibigkeit, bipolarer Störungen, Schizophrenie, Alopecia und Krebserkrankungen eine Rolle spielen.

Die insektizide Wirkung wurde für Xenobovid A, B und C bei Larven der Wachsmotte, einem Schädling in der Bienenzucht, nachgewiesen (Beispiel 10).

Die Anzucht von *Xenorhabdus bovienii,* so wie Aufreinigung von Xenobovid A, B und C aus Kulturen des Bakteriums und die Bestimmung der biologischen Aktivität werden in folgenden Beispielen beschrieben.

### 1) Biotechnologische Produktion von Xenoboviden aus Xenorhabdus bovienii

*X bovienii* wurde nach Akhurst et al. (1980, Morphological and functional dimorphism in Xenorhabdus spp., bacteria symbiotically associated with the insect pathogenic nematodes, Neoaplectana and Heterorhabditis. J. Gen. Microbiol. 121:303-309) aus dem Nematoden *Steinernema bibionis* isoliert. Die Kultivierung erfolgte für 36 Stunden in einem 20L-Fermenteransatz bei 20 °C (Johnigk et al., 2004, Liquid culture mass production of biocontrol nematodes, Heterorhabditis bacteriophora (Nematoda: Rhabditida): improved timing of dauer juvenile inoculation. Appl. Microbiol. Biotechnol. 64:651-658).

### 2) Isolierung von Xenoboviden aus der Bakterienkultur

Die Kulturlösung wurde geerntet und mit XAD-16N-Adsorberharz (Amberlite; 10 g/L Kulturlösung) für weitere 20 Stunden geschüttelt. Danach wurde das Harz mit Wasser gewaschen (5 mL/g XAD) und anschließend mit Methanol (2 mL/g XAD) eluiert. Der methanolische XAD-Extrakt wurde getrocknet, in Wasser (300 mL) resuspendiert und dreimal mit Ethylacetat (je 300 mL) extrahiert. Die vereinigten Ethylacetatphasen wurden getrocknet und durch Fast Centrifugal Partitioning Chromatography (FCPC; Kromaton) aufgetrennt. Dafür wurde ein zweiphasiges Lösungsmittelsystem (H₂O/MeOH/EtOAc/n-Heptan im Mischungsverhältnis 49:51:49:51) verwendet, wobei die obere Phase als stationäre Phase der Chromatographie diente. Die Umdrehungsgeschwindigkeit der FCPC betrug 1380 min⁻¹ und die Flussgeschwindigkeit 6 mL/min. Die Cyclodepsipeptid-haltigen Fraktionen, welche von 24 bis 63 min eluierten, wurden vereinigt, getrocknet und durch präparative HPLC weiter aufgereinigt:
Trennsäule: Phenomenex Luna C18, 21,2 x 250 mm, 5 µm
Lösungsmittel: Wasser + 0,1 % Ameisensäure (A), Acetonitril + 0,1 % Ameisensäure (B)
Gradient: 0 min - 50 % B, 8 min - 100 % B
Flussrate: 20 mL/min
Xenobovid A KW044 (15 mg), Xenobovid B KW12 (27 mg) und KW13 Xenobovid C (68 mg) eluierten nach 6,1, 8,0 und 8,5 min.

### 3) Antibakterielle Wirkung von Xenobovid A, B oder C

Xenobovid A, B bzw. C inhibierten das Wachstum des Gram-positiven Bakteriums *Staphylococcus xylosus* DSM 20267 um 83 %, 81 % bzw. 32 % in einer Konzentration von 65,3 µmol, 63,01 µmol bzw. 61,9 µmol. Die Testung der antimikrobiellen Aktivität erfolgte nach Lang et al. (2007, New pentaenes from the sponge-derived marine fungus Penicillium rugulosum: structure determination and biosynthetic studies. Tetrahedron 63:11844-11849).

Xenobovid B (63 µMol) und C (61,9 µMol) inhibierten das Wachstum des Gram-positiven Bakteriums *Bacillus subtilis* (DSM 347) um 29 % bzw. 32 %. Die Testung der antimikrobiellen Aktivität gegen *Bacillus subtilis* erfolgte nach Lang et al. (2007, New pentaenes from the sponge-derived marine fungus Penicillium rugulosum: structure determination and biosynthetic studies. Tetrahedron 63:11844-11849).

### 4) Antibakterielle Aktivität von Xenobovid A gegen Erreger von Pflanzenkrankheiten

Das phytopathogene Bakterium *Xanthomonas campestris* (DSM 2405), u.a. Erreger der Adernschwärze bei Kohl, wurde von Xenobovid A zu 89 % gehemmt. Die Testung der antimikrobiellen Aktivität gegen *Xanthomonas campestris* erfolgte nach Lang et al. (2007, New pentaenes from the sponge-derived marine fungus Penicillium rugulosum: structure determination and biosynthetic studies. Tetrahedron 63:11844-11849).

### 5) Antiproliferative Wirkung von Xenobovid A, B und C

Xenobovid A, B bzw. C hemmten die Proliferation aller getesteten 6 humanen Tumorzelllinien: GXF251L (Magentumor-Zelllinie), LXF529L (Lungentumor-Zelllinie), MAXF401NL (Brustkrebs-Zelllinie), MEXF462NL (Melanom-Zelllinie), PAXF1657L (Pankreastumor-Zelllinie) und RXF486L (Nierentumor-Zelllinie).

| **Tumorzelllinie** | **Xenobovid A** | **Xenobovid B** | **Xenobovid C** |
|---|---|---|---|
| | IC₅₀-Wert [µMol] | IC₅₀-Wert [µMol] | IC₅₀-Wert [µMol] |
| GXF251L | 1,6 | 2,5 | 3,0 |
| LXF529L | 8,5 | 9,7 | 7,8 |
| MAXF401NL | 2,1 | 3,2 | 8,5 |
| MEXF462NL | 2,7 | 5,3 | 9,4 |
| PAXF1657L | 3,5 | 2,9 | 10,3 |
| RXF486L | 12,0 | 14,5 | 7,6 |

Die antiproliferative Wirkung wurde nach Dengler et al. (1995, Development of a propidium iodide fluorescence assay for proliferation and cytoxicity assay. Anti-Cancer Drugs 6:522-532) ermittelt.

Die Proliferation der Darmtumor-Zelllinie HT29 wurde zu 7 % von Xenobovid A (65,3 µMol) bzw. 78 % von Xenobovid B (63 µMol) gehemmt. Xenobovid C wurde in einer Konzentration von 12,3 µMol getestet und zeigt eine Inhibierung der Vitalität der Zelllinie HT29 um 11 %. Diese Aktivitäten wurden nach einer 48-stündigen Inkubation mit dem Kristallviolett-Assay (Siegmund, D. et al., 2005, Death receptor-induced signalling pathways are differentially regulated by gamma interferon upstream of caspase 8 processing. Mol. Cell. Biol. 25:6363-6379) ermittelt.

### 6) Hemmung der Acetylcholinesterase durch Xenobovid A

Xenobovid A hemmt die Acetylcholinesterase mit einem IC₅₀-Wert von 53,6 µMol. Die Testdurchführung erfolgte nach Birman (1985, Determination of acetylcholinesterase activity by a new chemiluminescence assay with natural substrate. Biochemical Journal. 225:825-828).

### 7) Hemmung der Phosphodiesterase (PDE-4B2) durch Xenobovid A

Xenobovid A hemmt die Phosphodiesterase (PDE-4B2) in einer Konzentration von 163µg/mL um 46 %. Dieser Enzymtest wurde nach Hohmann et al. durchgeführt (2009, Caboxamycin, a new antibiotic of the benzoxazole family produced by the deep-sea strain Streptomyces sp. NTK 937. The Journal of Antibiotics. 62:99-104).

### 8) Hemmung der Protein Tyrosin Phosphatase 1B (PTP1B) durch Xenobovid A

Xenobovid A hemmt die Protein Tyrosin Phosphatase 1B (PTP1B) in einer Konzentration von 26,1 µMol um 59 %. Dieser Enzymtest wurde nach Hohmann et al. durchgeführt (2009, Caboxamycin, a new antibiotic of the benzoxazole family produced by the deep-sea strain Streptomyces sp. NTK 937. The Journal of Antibiotics. 62:99-104).

### 9) Hemmung der Glycogen Synthase Kinase 3beta durch Xenobovid A oder B

Xenobovid A bzw. B hemmten die Glycogen Synthase Kinase 3beta in einer Konzentration von 16,3 µMol bzw.15,6 µMol um 21 % bzw. 43 %. Die Durchführung erfolgte nach Baki et al. (2007, A high throughput luminescent assay for glycogen synthase kinase-3beta inhibitors. Assay and Drug Development Technologies. 5:75-83) unter Verwendung des Kinase-Glo Luminescent Kinase Assay Kit der Firma Promega.

### 10) Insektizide Wirkung Xenobovid A, B oder C

Die insektizide Wirkung wurde gegen die Larven der Wachsmotte, einem Schädling in der Bienenzucht, getestet. Der Zusatz von Xenobovid A, B oder C zum Futter führte zum Tod aller im Test eingesetzten Larven in 4 Wiederholungsversuchen. Zur Testung der oralen Toxizität wurden 1 mg Substanz/mL in einer 50%igen wässrigen Ethanol-Lösung gelöst. Jeweils 5 Larven wurden in eine Plastikbox (2 cm Durchmesser) überführt und mit 1 g Nahrungsgemisch (0,8 mL Substanzlösung, 0,2 mL Glycerin (86 %) und 1 g Weizenkleie) gefüttert. Die Kontrollgruppe wurde mit einem 1 g Nahrungsgemisch ohne Substanzzugabe (0,4 mL aq. dest., 0,4 mL Ethanol, 0,2 mL Glycerin (86 %) und 1 g Weizenkleie) gefüttert. Die Inkubation der Larven erfolgte bei 35 °C für 6 Tage. Im Anschluss wurden die toten Larven ausgezählt.

**Tabelle 1: NMR-Daten (600 MHz, DMSO) von Xenobovid A Farbloser amorpher Feststoff.**

| Optische Aktivität: [α]²⁵_{D} -1.6° (*c* 0.25, MeOH) | | | | | |
|---|---|---|---|---|---|
| HRESIMS: *m*/*z* 766.5075 [M+H]⁺ (berechnet für C₃₈H₆₈N₇O₉ 766.50785) | | | | | |
| **Position** | | **δ_{C}**, **mult.** | **δ_{H} (*J* in Hz)** | **COSY** | **HMBC** |
| *Thr* | 1 | 169.1, qC | - | - | - |
| | 2 | 55.0, CH | 4.57, *dd* (2.1, 9.3) | 3, NH | 1, 3, 4, *BA*-1 |
| | 3 | 70.6, CH | 5.22, *qd* (2.1, 6.3) | 2, 4 | 1, 4, *Ala₁*-1 |
| | 4 | 17.3, CH₃ | 1.16, *d* (6.3) | 3 | 2, 3 |
| | NH | - | 7.69, *d* (9.4) | 2 | 2 |
| *Ala₁* | 1 | 171.8, qC | - | - | - |
| | 2 | 48.5, CH | 4.15, *m* | 3, NH | 1, 3 |
| | 3 | 16.0, CH₃ | 1.29, *d* (6.9) | 2 | 1,2 |
| | NH | - | 8.16, *d* (5.3) | 2 | 2, *Leu₄*-1 |
| *Ala₂* | 1 | 172.2, qC | - | - | - |
| | 2 | 49.3, CH | 4.19, *m* (6.8) | 3, NH | 1, 3, *Thr*-1 |
| | 3 | 18.1, CH₃ | 1.26, *d* (7.2) | 2 | 1,2 |
| | NH | - | 8.00, *d* (6.4) | 2 | 2, *Thr-*1 |
| *Leu₁* | 1 | 173.5, qC | - | - | - |
| | 2 | 52.2, CH | 4.14, *m* | 3, NH | 1,3,4 |
| | 3 | 39.0, CH₂ | 1.58, *m* | 4 | 2,4 |
| | 4 | 24.3^{b}, CH | 1.62^{b}, *m* | 3, 5, 6 | 3, 5, 6 |
| | 5 | 23.1^{b}, CH₃ | 0.88^{b}, *m* | 4 | 3, 4, 6 |
| | 6 | 23.1^{b}, CH₃ | 0.88^{b}, *m* | 4 | 3, 4, 5 |
| | NH | - | 7.58, *d* (6.1) | 2 | *Ala₂-*1 |
| *Leu₂* | 1 | 172.0, qC | - | - | - |
| | 2 | 52.4, CH | 4.09, *m* | 3, NH | 1, 3, 4 |
| | 3 | 39.5, CH₂ | 1.62, *m* | 4 | 2, 4 |
| | 4 | 24.3^{b}, CH | 1.62^{b}, *m* | 3,5,6 | 3, 5, 6 |
| | 5 | 22.5^{b}, CH₃ | 0.92^{b}, *m* | 4 | 3, 4, 6 |
| | 6 | 22.5^{b}, CH₃ | 0.92^{b}, *m* | 4 | 3, 4, 5 |
| | NH | - | 7.53, *d* (6.8) | 2 | *Leu₁*-1 |
| *Leu₃* | 1 | 171.6, qC | - | - | - |
| | 2 | 51.5, CH | 4.14, *m* | 3, NH | 1, 3, 4 |
| | 3 | 39.5, CH₂ | 1.58, *m* | 4 | 2, 4 |
| | 4 | 24.2^{b}, CH | 1.62^{b}, *m* | 3, 5, 6 | 3, 5, 6 |
| | 5 | 21.7^{b}, CH₃ | 0.85^{b}, *m* | 4 | 3, 4, 6 |
| | 6 | 21.7^{b}, CH₃ | 0.85^{b}, *m* | 4 | 3, 4, 5 |
| | NH | - | 8.23, *d* (7.7) | 2 | *Leu₂*-1 |
| *Leu₄* | 1 | 172.0, qC | - | - | - |
| | 2 | 50.6, CH | 4.26, *m* | 3, NH | 1, 3, 4 |
| | 3 | 39.6, CH₂ | 1.51, *m* | 4 | 2, 4 |
| | 4 | 24.1^{b}, CH | 1.62^{b}, *m* | 3, 5, 6 | 3, 5, 6 |
| | 5 | 20.7^{b}, CH₃ | 0.81⁶, *m* | 4 | 3, 4, 6 |
| | 6 | 20.7^{b}, CH₃ | 0.81^{b}, *m* | 4 | 3, 4, 5 |
| | NH | - | 7.51, *d* (8.9) | 2 | *Leu₃*-1 |
| *BA*^{a} | 1 | 172.4, qC | - | - | - |
| | 2a | 44.5, CH₂ | 2.19, *m* | 2b, 3 | 1, 3, 4 |
| | 2b | 44.5, CH₂ | 2.11, *m* | 2a, 3 | 1, 3, 4 |
| | 3 | 25.6, CH₂ | 2.00, *m* | 2a, 2b, 4 | 1, 2, 4 |
| | 4 | 13.9, CH₃ | 0.90, *m* | 3 | 3 |

| | | | | | |
|---|---|---|---|---|---|
| ^{a} BA: Buttersäure ^{b} die Zuordnung kann ausgetauscht werden | | | | | |

**Tabelle 2: NMR-Daten (600 MHz, DMSO) von Xenobovid B Farbloser amorpher Feststoff.**

| Optische Aktivität: [α]²⁵_{D} -1.6° (*c* 0.25, MeOH) | | | | | |
|---|---|---|---|---|---|
| HRESIMS: *m*/*z* 794.5380 [M+H]⁺ (berechnet für C₄₀H₇₂N₇O₉ 794.53915) | | | | | |
| **Position** | | **δ_{C}**, **mult.** | **δ_{H} (*J* in Hz)** | **COSY** | **HMBC** |
| *Thr* | 1 | 169.2, qC | - | - | - |
| | 2 | 55.0, CH | 4.55, *dd* (2.1, 9.2) | 3, NH | 1, 3, 4, *MPA*-1 |
| | 3 | 70.6, CH | 5.21, *qd* (2.1, 6.3) | 2, 4 | 1, 4, *Ala₁*-1 |
| | 4 | 17.0, CH₃ | 1.15, *d* (6.3) | 3 | 2, 3 |
| | NH | - | 7.67, *d* (9.3) | 2 | *2, MPA*-1 |
| *Ala₁* | 1 | 171.7, qC | - | - | - |
| | 2 | 48.5, CH | 4.14, *m* | 3, NH | 1, 3 |
| | 3 | 16.0, CH₃ | 1.29, *d* (7.2) | 2 | 1, 2 |
| | NH | - | 8.13, *d* (5.5) | 2 | 2, *Leu₄*-1 |
| *Ala₂* | 1 | 172.3, qC | - | - | - |
| | 2 | 49.2, CH | 4.19, *m* (6.7) | 3, NH | 1, 3, *Thr*-1 |
| | 3 | 17.9, CH₃ | 1.25, *d* (7.2) | 2 | 1, 2 |
| | NH | - | 7.96, *d* (6.2) | 2 | 2, *Thr*-1 |
| *Leu₁* | 1 | 172.0, qC | - | - | 1, 3, |
| | 2 | 52.0, CH | 4.11, *m* | 3, NH | 1, 3, 4 |
| | 3 | 39.0, CH₂ | 1.52, *m* | 4 | 2, 4 |
| | 4 | 24.2^{b}, CH | 1.62^{b}, *m* | 3,5,6 | 3, 5, 6 |
| | 5 | 23.0^{b}, CH₃ | 0.88^{b}, *m* | 4 | 3, 4, 6 |
| | 6 | 23.0^{b}, CH₃ | 0.88^{b}, *m* | 4 | 3, 4, 5 |
| | NH | - | 7.56, *d* (6.1) | 2 | *Ala₂*-1 |
| *Leu₂* | 1 | 171.9, qC | - | - | - |
| | 2 | 52.3, CH | 4.08, *m* | 3, NH | 1, 3, 4 |
| | 3 | 39.5, CH₂ | 1.62, *m* | 4 | 2, 4 |
| | 4 | 24.2^{b}, CH | 1.62^{b}, *m* | 3, 5, 6 | 3, 5, 6 |
| | 5 | 22.5^{b}, CH₃ | 0.92^{b}, *m* | 4 | 3, 4, 6 |
| | 6 | 22.5^{b}, CH₃ | 0.92^{b}, *m* | 4 | 3, 4, 5 |
| | NH | - | 7.53, *d* (6.8) | 2 | *Leu₁*-1 |
| *Leu₃* | 1 | 171.5, qC | - | - | - |
| | 2 | 51.5, CH | 4.13 , *m* | 3, NH | 1, 3, 4 |
| | 3 | 39.5, CH₂ | 1.56, *m* | 4 | 2, 4 |
| | 4 | 24.1^{b}, CH | 1.62^{b}, *m* | 3, 5, 6 | 3, 5, 6 |
| | 5 | 21.7^{b}, CH₃ | 0.85^{b}, *m* | 4 | 3, 4, 6 |
| | 6 | 21.7^{b}, CH₃ | 0.85^{b}, *m* | 4 | 3, 4, 5 |
| | NH | - | 8.24, *d* (7.9) | 2 | *Leu₂*-1 |
| *Leu₄* | 1 | 171.9, qC | - | - | - |
| | 2 | 50.5, CH | 4.25 , *m* | 3, NH | 1, 3, 4 |
| | 3 | 39.6, CH₂ | 1.50, *m* | 4 | 2, 4 |
| | 4 | 24.0^{b}, CH | 1.62^{b}, *m* | 3, 5, 6 | 3, 5, 6 |
| | 5 | 20.7^{b}, CH₃ | 0.81^{b}, *m* | 4 | 3, 4, 6 |
| | 6 | 20.7^{b}, CH₃ | 0.81^{b}, *m* | 4 | 3, 4, 5 |
| | NH | - | 7.50, *d* (8.7) | 2 | *Leu₃*-1 |
| *MPA*^{a} | 1 | 172.6, qC | - | - | - |
| | 2a | 35.0, CH₂ | 2.28, *m* | 2b, 3 | 1, 3, 4 |
| | 2b | 35.0, CH₂ | 2.19, *m* | 2a, 3 | 1, 3, 4 |
| | 3 | 24.8, CH | 1.51, *m* | 2a, 2b, 4, 6 | 1, 2, 4, 6 |
| | 4 | 30.7, CH₂ | 1.24, *m* | 3, 5 | 3, 5 |
| | 5 | 13.1, CH₃ | 0.85, *m* | 4 | 4 |
| | 6 | 21.4, CH₃ | 1.28, *m* | 3 | 3 |

| | | | | | |
|---|---|---|---|---|---|
| ^{a} MPA: 3-Methylpentansäure ^{b} die Zuordnung kann ausgetauscht werden | | | | | |

**Tabelle 3: NMR-Daten (600 MHz, DMSO) von Xenobovid C Farbloser amorpher Feststoff.**

| Optische Aktivität: [α]²⁵_{D} -2.4° (*c* 0.25, MeOH) | | | | | |
|---|---|---|---|---|---|
| HRESIMS: *m*/*z* 808.5545 [M+H]⁺ (berechnet für C₄₁H₇₄N₇O₉ 808.55480) | | | | | |
| **Position** | | **δ_{C}**, **mult.** | **δ_{H} (*J* in Hz)** | **COSY** | **HMBC** |
| *Thr* | 1 | 169.0, qC | - | - | - |
| | 2 | 55.1, CH | 4.55, *dd* (2.0, 9.3) | 3, NH | 1, 3, 4, *MHA*-1 |
| | 3 | 70.6, CH | 5.22, *qd* (2.0, 6.3) | 2, 4 | 1, 4, *Ala₁*-1 |
| | 4 | 177.1, CH₃ | 1.16, *d* (6.3) | 3 | 2, 3 |
| | NH | - | 7.69, *d* (9.3) | 2 | 2, *MHA*-1 |
| *Ala₁* | 1 | 171.8, qC | - | - | - |
| | 2 | 48.5, CH | 4.15, *m* | 3, NH | 1, 3 |
| | 3 | 16.0, CH₃ | 1.29, *d* (7.1) | 2 | 1, 2 |
| | NH | - | 8.14, *d* (5.4) | 2 | 2, *Leu₄*-1 |
| *Ala₂* | 1 | 172.4, qC | - | - | - |
| | 2 | 49.4, CH | 4.19, *m* (6.7) | 3, NH | 1, 3, *Thr*-1 |
| | 3 | 18.0, CH₃ | 1.25, *d* (7.2) | 2 | 1, 2 |
| | NH | - | 7.97, *d* (6.2) | 2 | 2, *Thr*-1 |
| *Leu₁* | 1 | 172.1, qC | - | - | - |
| | 2 | 52.1, CH | 4.13, *m* | 3, NH | 1, 3, 4 |
| | 3 | 39.0, CH₂ | 1.53, *m* | 4 | 2, 4 |
| | 4 | 24.2^{b}, CH | 1.63^{b}, *m* | 3, 5, 6 | 3, 5, 6 |
| | 5 | 23.0^{b}, CH₃ | 0.88^{b}, *m* | 4 | 3, 4, 6 |
| | 6 | 23.0^{b}, CH₃ | 0.88^{b}, *m* | 4 | 3, 4, 5 |
| | NH | - | 7.58, *d* (6.0) | 2 | *Ala₂*-1 |
| *Leu₂* | 1 | 172.0, qC | - | - | - |
| | 2 | 52.4, CH | 4.09, *m* | 3, NH | 1, 3, 4 |
| | 3 | 39.5, CH₂ | 1.63, *m* | 4 | 2, 4 |
| | 4 | 24.2^{b}, CH | 1.63^{b}, *m* | 3,5,6 | 3, 5, 6 |
| | 5 | 22.5^{b}, CH₃ | 0.92^{b}, *m* | 4 | 3, 4, 6 |
| | 6 | 22.5^{b}, CH₃ | 0.92^{b}, *m* | 4 | 3, 4, 5 |
| | NH | - | 7.54, *d* (6.7) | 2 | *Leu₁*-1 |
| *Leu₃* | 1 | 171.6, qC | - | - | - |
| | 2 | 51.6, CH | 4.14, *m* | 3, NH | 1, 3, 4 |
| | 3 | 39.5, CH₂ | 1.59, *m* | 4 | 2, 4 |
| | 4 | 24.1^{b}, CH | 1.63^{b}, *m* | 3, 5, 6 | 3, 5, 6 |
| | 5 | 21.7^{b}, CH₃ | 0.85^{b}, *m* | 4 | 3, 4, 6 |
| | 6 | 21.7^{b}, CH₃ | 0.85^{b}, *m* | 4 | 3, 4, 5 |
| | NH | - | 8.24, *d* (7.7) | 2 | *Leu₂*-1 |
| *Leu₄* | 1 | 172.0, qC | - | - | - |
| | 2 | 50.6, CH | 4.26, *m* | 3, NH | 1, 3, 4 |
| | 3 | 39.6, CH₂ | 1.51, *m* | 4 | 2, 4 |
| | 4 | 24.0⁶, CH | 1.63^{b}, *m* | 3, 5, 6 | 3, 5, 6 |
| | 5 | 20.7^{b}, CH₃ | 0.82^{b}, *m* | 4 | 3, 4, 6 |
| | 6 | 20.7^{b}, CH₃ | 0.82^{b}, *m* | 4 | 3, 4, 5 |
| | NH | - | 7.51, *d* (8.8) | 2 | *Leu₃*-1 |
| *MHA*^{a} | 1 | 172.7, qC | - | - | - |
| | 2a | 35.3, CH₂ | 2.29, *m* | 2b, 3 | 1, 3, 4 |
| | 2b | 35.3, CH₂ | 2.20, *m* | 2a, 3 | 1, 3, 4 |
| | 3 | 23.1, CH | 1.51, *m* | 2a, 2b, 4, 7 | 1, 2, 4, 7 |
| | 4 | 37.9, CH₂ | 1.15, *m* | 3, 5 | 3, 5 |
| | 5 | 27.1, CH₂ | 1.51, *m* | 4,6 | 4, 6 |
| | 6 | 23.1, CH₃ | 0.85, *m* | 5 | 5, 7 |
| | 7 | 22.4, CH₃ | 0.88, *m* | 3 | 3 |

| | | | | | |
|---|---|---|---|---|---|
| ^{a} MHA: 3-Methylhexansäure ^{b} die Zuordnung kann ausgetauscht werden | | | | | |

## Patentansprüche

1. Verbindung der allgemeinen Struktur 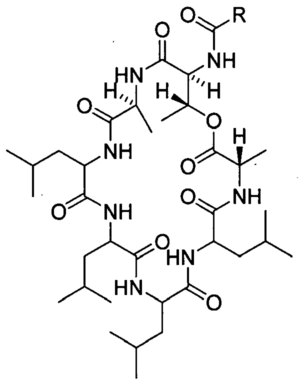 wobei R ein Wasserstoff-Atom (H) und/oder ein unsubstituiertes, monosubstituiertes oder polysubstituiertes C₁-C₂₀-Alkyl ist, wobei das Alkyl gerade, verzweigt, zyklisch und/oder teilweise ungesättigt ist, oder ein unsubstituierter, monosubstituierter oder mehrfach substituierter Phenylrest ist.

2. Verbindung nach Anspruch 1, **dadurch gekennzeichnet, dass** die Substituenten von R ausgewählt sind aus der Gruppe bestehend aus einem linearen oder verzweigten Alkylrest, einer Acylgruppe, einem Halogenrest, einer unsubstituierten oder alkylsubstituierten Aminogruppe, einer Hydroxylgruppe, einer Ethergruppe und einer freien sowie mit Alkylgruppe veresterten oder amidierten Carboxylgruppe, wobei die Acylgruppe bevorzugt eine Formyl-, Acetyl-, Trichloracetyl-, Fumaryl-, Maleyl-, Succinyl-, Benzoyl-, oder eine verzweigte oder heteroatom- oder arylsubstituierte Acylgruppe ist.

3. Verbindung nach Anspruch 1 mit der Formel 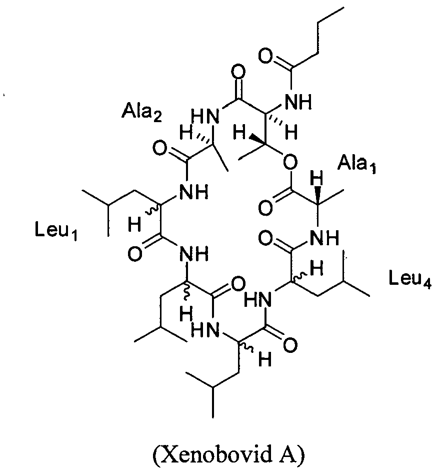 einschließlich deren Diastereomere, oder mit der Formel 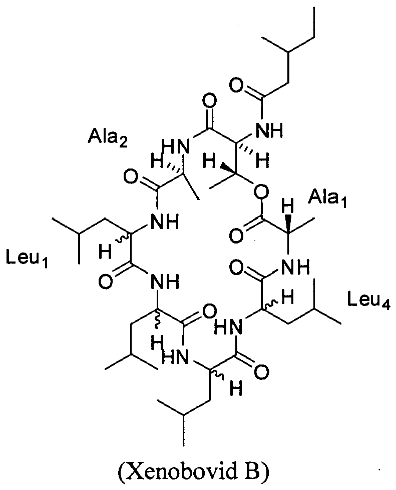 einschließlich deren Diastereomere, oder mit der Formel 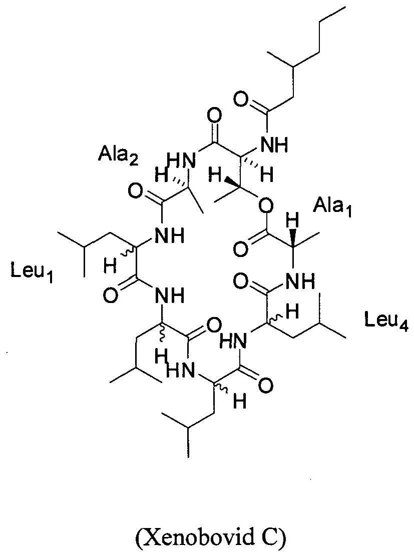 einschließlich deren Diastereomere.

4. Verfahren zur Herstellung einer Verbindung nach einem der vorhergehenden Ansprüche, **gekennzeichnet durch** die Schritte:
- Kultivieren eines Bakteriums der Gattung *Xenorhabdus* auf an sich bekannte Weise und
- Isolieren der Verbindung aus dem Kulturmedium und/oder dem Bakterium.

5. Verfahren nach Anspruch 4, **dadurch gekennzeichnet, dass** das Bakterium *Xenorhabdus bovienii* ist.

6. Substanz nach einem der Ansprüche 1 bis 3 zur Verwendung als Antitumormittel, bevorzugt zur Behandlung von und zur Vorbeugung vor Melanom, Magentumor-, Lungentumor-, Brustkrebs-, Pankreastumor-, Nierentumor- und Darmtumorerkrankungen.

7. Substanz nach einem der Ansprüche 1 bis 3 zur Verwendung als Mittel zur Behandlung und Prävention von neurologischen Erkrankungen, insbesondere Myasthenia Gravis oder Alzheimer, oder akuten und chronischen neurodegenerativen Erkrankungen.

8. Substanz nach einem der Ansprüche 1 bis 3 zur Verwendung als Mittel zur Entzündungshemmung und Prävention, insbesondere bei Rhinitis oder entzündlichen Lungenerkrankungen ausgewählt aus der Gruppe umfassend Asthma, chronisch obstruktive Bronchitis und Lungenemphysem.

9. Substanz nach einem der Ansprüche 1 bis 3 zur Verwendung als Psychopharmakon, insbesondere als Antidepressivum oder zur Behandlung von bipolarer Störung oder Schizophrenie.

10. Substanz nach einem der Ansprüche 1 bis 3 zur Verwendung bei Behandlung von Alopecia oder AIDS oder als Antibiotikum.

11. Substanz nach einem der Ansprüche 1 bis 3 zur Verwendung als Mittel zur Behandlung und Prävention von Diabetes oder Fettleibigkeit.

12. Substanz nach einem der Ansprüche 1 bis 3 zur Verwendung als Insektizid.

13. Nicht-therapeutische Verwendung einer Substanz nach einem der Ansprüche 1 bis 3 als Insektizid.

14. Verwendung einer Substanz nach einem der Ansprüche 1 bis 3 als Pflanzenschutzmittel.

15. In vitro-Verwendung einer Substanz nach einem der Ansprüche 1 bis 3 als Acetylcholinesterase-Inhibitor, als Phosphodiesterase-Inhibitor, als Protein Tyrosin Phosphatase-Inhibitor oder als Glycogen Synthase Kinase 3beta-Inhibitor.

## Claims

1. A composition having the general structure 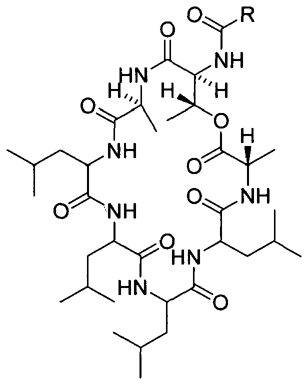 wherein R is a hydrogen atom (H) and/or an unsubstituted, monosubstituted or polysubstituted C₁-C₂₀ alkyl, wherein the alkyl is straight, branched, cyclic and/or partially unsaturated, or is an unsubstituted, monosubstituted or polysubstituted phenyl residue.

2. A composition according to claim 1, wherein the substituents of R are selected from the group consisting of a linear or branched alkyl residue, an acyl residue, a halogen residue, an unsubstituted or alkyl-substituted amino group, a hydroxyl residue, an ether residue and a carboxyl residue which is free or esterified with an alkyl residue or amidated, wherein the acyl residue preferably is a formyl-, acetyl-, trichloroacetyl-, fumaryl-, maleyl-, succinyl-, benzoyl-, or an acyl residue which is branched or substituted with a heteroatom or aryl..

3. A composition according to claim 1 having the formula 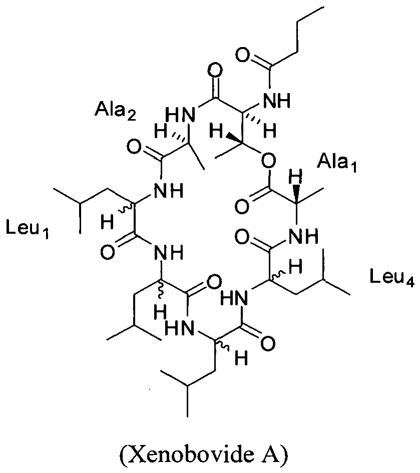 including its diastereomers, or having the formula 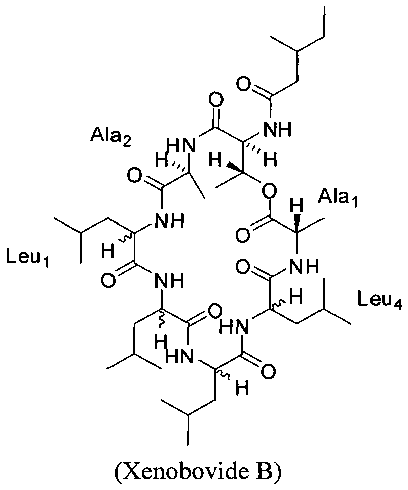 including its diastereomers, or having the formula 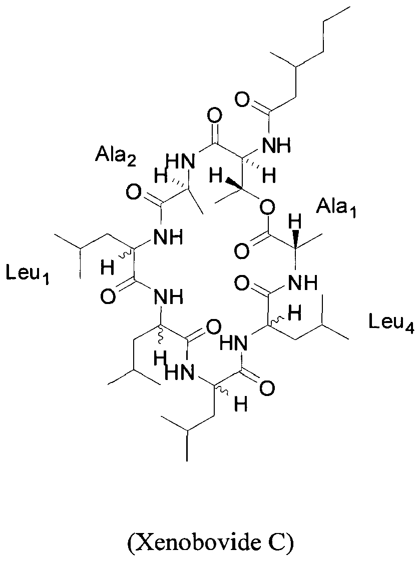 including its diastereomers.

4. A method for preparing a composition according to any of the preceding claims, comprising the steps of:
- cultivating a bacterium from the genus *Xenorhabdus* in a matter known per se, and
- isolating the composition from the culture medium and/or the bacterium.

5. Method according to claim 4, wherein the bacterium is *Xenorhabdus bovienii.*

6. A substance according to any of claims 1 to 3 for use as an anti tumor agent, preferably for treatment and for prevention of melanoma, gastric tumor, lung tumor, breast cancer, pancreatic tumor, renal tumor and gut tumor diseases.

7. A substance according to any of claims 1 to 3 for use as an agent for treatment and for prevention of neurological diseases, in particular, myasthenia gravis or Alzheimer's disease, or acute or chronic neurodegenerative diseases.

8. A substance according to any of claims 1 to 3 for use as an agent for inhibiting and preventing inflammation, in particular in rhinitis or inflammatory lung diseases selected from the group comprising asthma, chronic obstructive pulmonary disease and pulmonary emphysema.

9. A substance according to any of claims 1 to 3 for use as a psychotropic drug, in particular as an antidepressant or for treatment of bipolar disorder or schizophrenic disorder.

10. A substance according to any of claims 1 to 3 for use in treatment of alopecia or AIDS or as an antibiotic.

11. A substance according to any of claims 1 to 3 for use as an agent for treatment and prevention of diabetes and adipositas.

12. A substance according to any of claims 1 to 3 for use as an insecticide.

13. Non-therapeutic use of a substance according to any of claims 1 to 3 as an insecticide.

14. Use of a substance according to any of claims 1 to 3 as a plant protecting agent.

15. *In vitro* use of a substance according to any of claims 1 to 3 as an inhibitor of acetylcholineesterase, as an inhibitor of phosphodiesterase, as an inhibitor of protein tyrosin phosphatase or as an inhibitor of glycogen synthase kinase 3beta.

## Revendications

1. Composé de formule générale 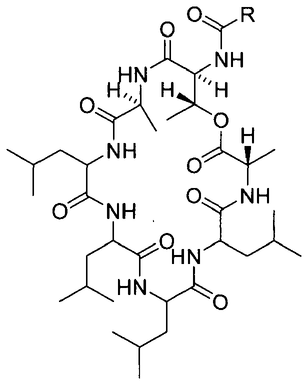 dans laquelle R représente un atome d'hydrogène (H) et/ou un groupe alkyle en C₁-C₂₀ sans aucun substituant ou porteur d'un substituant ou de plusieurs substituants, lequel groupe alkyle est linéaire, ramifié ou cyclique et/ou partiellement insaturé, ou un groupe phényle sans aucun substituant ou porteur d'un substituant ou de plusieurs substituants.

2. Composé conforme à la revendication 1, **caractérisé en ce que** les substituants du groupe représenté par R sont choisis dans l'ensemble formé par un groupe alkyle linéaire ou ramifié, un groupe acyle, un atome d'halogène, un groupe amino sans substituant ou porteur de substituant(s) alkyle, un groupe hydroxyle, un groupe de type éther, et un groupe carboxyle, libre ou bien estérifié avec un groupe alkyle ou amidifié, étant entendu que le groupe acyle est de préférence un groupe formyle, acétyle, trichloroacétyle, fumaryle, maléyle, succinyle ou benzoyle ou un groupe acyle ramifié ou doté d'un substituant aryle ou à hétéroatome.

3. Composé conforme à la revendication 1, de formule 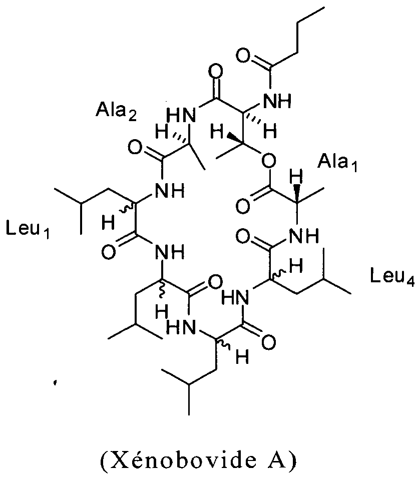 y compris ses diastéréoisomères, ou de formule 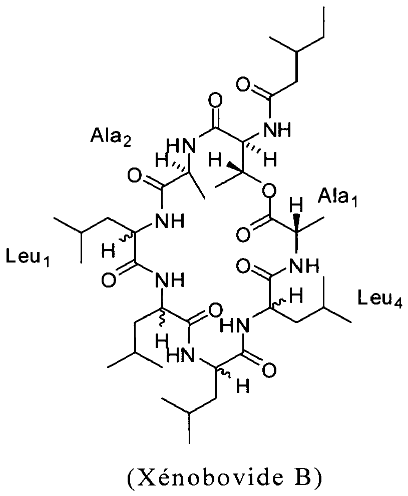 y compris ses diastéréoisomères, ou de formule 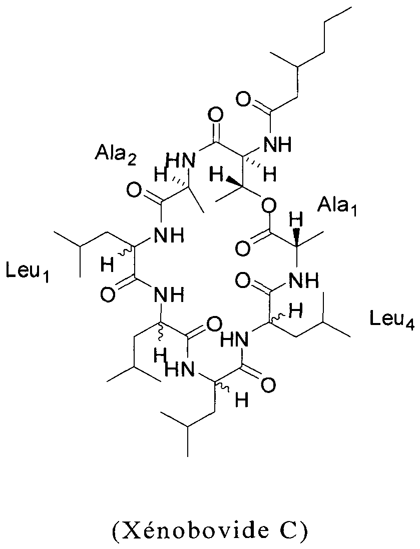 y compris ses diastéréoisomères.

4. Procédé de préparation d'un composé conforme à l'une des revendications précédentes, **caractérisé par** les étapes suivantes :
- cultiver une bactérie du genre *Xenorhabdus,* d'une manière connue en soi,
- et isoler le composé, à partir du milieu de culture et/ou à partir des bactéries.

5. Procédé conforme à la revendication 4, **caractérisée en ce que** la bactérie est *Xenorhabdus bovienii.*

6. Substance conforme à l'une des revendications 1 à 3, pour utilisation en tant qu'agent anti-tumoral, de préférence pour le traitement ou la prévention d'un mélanome ou d'un cancer de l'estomac, du poumon, du sein, du pancréas, du rein ou de l'intestin.

7. Substance conforme à l'une des revendications 1 à 3, pour utilisation en tant qu'agent pour le traitement ou la prévention de maladies neurologiques, en particulier la myasthénie grave ou la maladie d'Alzheimer, ou de maladies neurodégénératives aiguës ou chroniques.

8. Substance conforme à l'une des revendications 1 à 3, pour utilisation en tant qu'agent servant à inhiber ou prévenir les inflammations, en particulier pour les rhinites ou les maladies pulmonaires inflammatoires choisies dans l'ensemble comprenant l'asthme, la bronchite chronique obstructive et l'emphysème pulmonaire.

9. Substance conforme à l'une des revendications 1 à 3, pour utilisation en tant qu'agent psychopharmaceutique, en particulier comme antidépresseur ou pour le traitement du trouble bipolaire ou de la schizophrénie.

10. Substance conforme à l'une des revendications 1 à 3, pour utilisation dans le traitement de l'alopécie ou du sida, ou comme antibiotique.

11. Substance conforme à l'une des revendications 1 à 3, pour utilisation en tant qu'agent pour le traitement ou la prévention du diabète ou de l'obésité.

12. Substance conforme à l'une des revendications 1 à 3, pour utilisation en tant qu'insecticide.

13. Utilisation non-thérapeutique d'une substance conforme à l'une des revendications 1 à 3, en tant qu'insecticide.

14. Utilisation d'une substance conforme à l'une des revendications 1 à 3 en tant qu'agent phyto-protecteur.

15. Utilisation *in vitro* d'une substance conforme à l'une des revendications 1 à 3, en tant qu'inhibiteur de l'acétyl-choline estérase, en tant qu'inhibiteur de phosphodiestérase, en tant qu'inhibiteur de la protéine tyrosine phosphatase, ou en tant qu'inhibiteur de la glycogène synthétase kinase-3β.
